(19) 

Europäisches
Patentamt
European
Patent Office
Office européen
des brevets

(11) **EP 4 079 294 A2**

(12) **EUROPEAN PATENT APPLICATION**
published in accordance with Art. 153(4) EPC

(43) Date of publication:
**26.10.2022 Bulletin 2022/43**

(21) Application number: **20903282.0**

(22) Date of filing: **10.12.2020**

(51) International Patent Classification (IPC):
*A61K 9/08* [(2006.01)]          *C07K 16/28* [(2006.01)]
*A61K 47/02* [(2006.01)]         *A61K 47/18* [(2017.01)]
*A61K 47/26* [(2006.01)]         *A61K 9/19* [(2006.01)]
*A61K 31/198* [(2006.01)]        *A61P 1/00* [(2006.01)]

(52) Cooperative Patent Classification (CPC):
(C-Sets available)
**A61K 9/0019; A61K 9/08; A61K 9/19;
A61K 31/198; A61K 47/02; A61K 47/183;
A61K 47/26; A61P 1/00; C07K 16/2863;**
C07K 2317/94                                    (Cont.)

(86) International application number:
**PCT/CU2020/050007**

(87) International publication number:
**WO 2021/121444 (24.06.2021 Gazette 2021/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.12.2019 CU 20190104**

(71) Applicant: **Centro de Inmunologia Molecular
La Habana 11600 (CU)**

(72) Inventors:
• **HERNÁNDEZ TERRERO, Yaiko Saddan
La Habana 10400 (CU)**

• **FERNÁNDEZ SÁEZ, Olga Lidea
La Habana 10700 (CU)**
• **SANTO TOMÁS POMPA, Julio Felipe
La Habana 10900 (CU)**
• **CEDEÑO ARIAS, Mercedes
La Habana 11300 (CU)**
• **RASHIDA DE LA LUZ HERNÁNDEZ, Kathya
La Habana 10400 (CU)**
• **BOGGIANO AYO, Tammy
Playa La Habana 11300 (CU)**
• **LEÓN MONZÓN, Kalet
La Habana 17100 (CU)**
• **CASTILLO VITLOCH, Adolfo
La Habana 17100 (CU)**

(74) Representative: **Capitán García, Maria Nuria
Felipe IV no. 10, bajo iz.
28014 Madrid (ES)**

(54) **STABLE AND HIGHLY CONCENTRATED FORMULATION OF THE ANTIBODY NIMOTUZUMAB**

(57)      The present invention is related to the branches of Biotechnology and Medicine. It particularly describes highly concentrated and stable pharmaceutical formulations of the humanized monoclonal antibody nimotuzumab at a concentration within the range from 50 to 200 mg/mL. The low viscosity of these solutions allow for their administration by the subcutaneous or intramuscular routes in the treatment of cancer. These formulations are stable in both their liquid and lyophilized forms.

**FIGURE 7**

EP 4 079 294 A2

**(Cont. next page)**

(52) Cooperative Patent Classification (CPC): (Cont.)

C-Sets
**A61K 31/198, A61K 2300/00**

**Description**

**SCOPE OF THE TECHNIQUE**

The present invention is related to the branches of Biotechnology and Medicine, especially to the obtainment of a highly concentrated and stable formulation of the humanized monoclonal antibody nimotuzumab intended for subcutaneous (SC) or intramuscular (IM) administration as cancer treatment.

**BACKGROUND**

Nimotuzumab is a humanized IgG1 isotype monoclonal antibody (mAb) that recognizes the human epidermal growth factor receptor Her1. It was obtained by cloning the DNA of the hypervariable regions of murine origin mAb ior egf/r3 and the frames of the variable regions and constant regions of the heavy and light chains of human origin (REI and NEWM, respectively) (Mateo, C. et al. (1997), Immunotechnology 3: 71-81). The efficacy of nimotuzumab has been demonstrated in clinical trials, in patients with head and neck tumors (Crombet, T. et al. (2004) J Clin Oncol. 22: 1646-1654), glioma (Ramos, T. et al. (2006) Cancer Biol Ther. 5: 375-379; MacDonald, T. et al. (2011) Neuro Oncol. 13: 1049-1058) and esophageal tumor (Ramos-Suzarte, M. et al. (2012) Cancer Biology & Therapy 13: 600-605). This antibody (Ab) is currently being tested in phase III clinical trials in nasopharyngeal cancer, locally advanced esophageal cancer, and squamous cell carcinoma of the esophagus (Galluzzi, L. et al. (2012) OncoImmunology. 1: 28-37). The administration of mAb nimotuzumab is performed by intravenous (IV) route as infusion using a liquid formulation in where said mAb is at a concentration of 5 mg/mL (https://www.cecmed.cu/registro/rcp/cimaher-nimotuzumab). This administration is performed by dissolving four vials of nimotuzumab at a concentration of 5 mg/mL in a bag of 0.9% NaCl solution. The composition of the commercial formulation of nimotuzumab also comprises a sodium phosphate buffer at a concentration of 15 mM with pH at approximately 7, sodium chloride at 150 mM and polysorbate 80 at 0.02% (Revista Cubana de Farmacia. 2012; 46 (3): 379-380). Nimotuzumab in the clinical setting is administered at a dose of 200 mg (400 mg in pancreatic cancer) once a week for six weeks, combined with radiation therapy and chemotherapy. Then maintenance doses of 200mg are administered every 15 days until the clinical condition of the patient allows it. Hence the importance of having an administration route that facilitates this chronic use of nimotuzumab in patients outside the hospital setting.

As it is known, IV administration has some disadvantages such as the long period of time required to perform the administration, the need for qualified personnel trained in the administration procedure, the risk of infection and severe adverse reactions (Michael F. Haller (2007) Pharmaceutical Technology, 31 (10): 118-132).

Due to this, there is an increased trend worldwide towards SC administration of mAbs (Viola M, Sequeira J, et al. (2018) J Control Release; 286: 301-314) since this route offers advantages such as a reduction in the time required to perform the administration and the volumes administered, a reduction of infection risks and even the possibility of self-administration of the medicine by the patient, since manipulating and administering a single vial with the required quantity is easier (Haller MF (2007) Pharmaceutical Technology, 31 (10): 118 -132). IM administration also has advantages as compared to IV administration, such as lower treatment costs and less intensive care required. On the other hand, the risk of infection is lower and the administration time is shorter with the IM route than the IV route. Additionally, it is known that there is a lower chance of occurrence of adverse effects and of overdose when administering a medicine by the IM route. It should also be noted that the absorption and bioavailability of the medicine after IM administration is quite high, with values almost as high as the ones reported when using the IV route. (Jing-fen Jin et al., Patient Preference and Adherence, 2015, 9: 923-942). Among the mAbs that are currently used in the clinic in SC formulations are trastuzumab (US 9,345,661) and rituximab (US 10,280,227), in both formulations the hyaluronidase enzyme is used to increase the injection volume to more than 1.5 mL and achieve a good biodistribution of the mAb thus ensuring that the dose necessary to exert therapeutic effect is reached (Shpilberg, O. and C. Jackisch. (2013) British Journal of Cancer. 109: 1556-1561).

Another of the highly concentrated formulations that use the hyaluronidase enzyme is the one described in patent application CN107898756A. In this patent, a formulation of the nimotuzumab mAb at high concentrations for SC or IM use is claimed. As reported in the patent, the presence of hyaluronidase in this formulation is necessary to ensure the required permeability is achieved.

There are previous reports of highly concentrated formulations of mAbs against the epidermal growth factor such as the one described in patent application WO2011080209, in where a high concentration liquid formulation obtained by ultrafiltration that comprises an anti-EGFR mAb is claimed. Although in this patent it is stated that the claimed formulation can be used for nimotuzumab mAb, it does not provide practical evidence supporting this claim. In this patent application a concentration of the mAb ranging from 1 mg/ml to 200 mg/ml is claimed, however, only a concentration of up to 50 mg/ ml of the mAb hu-ICR62 is reached. Furthermore, the ranges of excipients and concentrations claimed in this patent application are too wide because they comprise almost all the most commonly used excipients and concentrations in the state of the art. In the present invention, it was demonstrated that some combinations of excipients and concentrations included in the range claimed in WO2011080209 were not the most stable or the best formulation options for nimotuzumab mAb.

As the main example of a mAb administered by IM route is palivuzumab mAb. This mAb is presented as a lyophilized formulation that, after being reconstituted in water, has palivuzumab mAb at a concentration of 100 mg/mL in addition to excipients such as histidine, glycine and mannitol and is used in 15 mg/kg doses for the treatment of respiratory syncytial virus.

Achieving a formulation of the mAb that maintains stability, low turbidity and viscosity is a challenge that requires an adequate screening methodology. The challenge of finding the right combination of excipients and conditions required for a mAb to meet the abovestated requirements at a high concentration is not trivial, since up to now it is not possible to predict a priori which combination will be the most effective for a specific mAb since each one is different and behaves differently in terms of their main degradation mechanisms (Manning MC et al. (2018) Advances in Protein Chemistry and Structural Biology, 112: 1-59; Viola M, Sequeira J, et al. (2018) J Control Release; 286 : 301-314). The inventors of the present invention found formulations for nimotuzumab mAb different from the ones reported so far that have a specific combination of excipients that guarantees the stability, low turbidity and low viscosity of the mAb. These formulations are also less complex since they do not require the use of the hyaluronidase enzyme to guarantee the stability and permeability of the SC formulation. With these specific formulations, concentrations between 100 and 180 mg/mL of nimotuzumab are also achieved, thus allowing for the mAb dose to be increased without increasing the volume of administration. The administration of these formulations by SC or IM route facilitates their use when a chronic administration is required, at doses between 200 mg and 360 mg in the case of the SC route and between 200-720 mg in that of the IM route, with the additional advantage that they can be self- administered by the patient.

## BRIEF DESCRIPTION OF THE INVENTION

In one embodiment, the object of the present invention are highly concentrated and stable pharmaceutical formulations of nimotuzumab monoclonal Ab that comprise nimotuzumab mAb at a concentration range between 100 and 210 mg/mL, a buffer substance at a range between 5 and 30 mM, with a pH value of 6.5 $\pm$ 0.5, a surfactant at a range between 0.02 and 0.06%, an amino acid or a mixture thereof at a range between 30 and 150mM and optionally a carbohydrate as stabilizer at a range between 2 and 6%.

In particular, the buffer substance of said formulations is selected from histidine buffer and sodium phosphate. The surfactant is selected from polysorbate 20 and polysorbate 80. The amino acids are selected from L-methionine and glycine. The carbohydrate used in the formulation is sucrose.

More particularly, the above formulations can be in liquid or lyophilized form. The liquid formulation at a concentration of 150 mg/mL has a viscosity $\leq$ 5cP.

It is a further object of the present invention the use of the pharmaceutical formulations described above in the treatment of cancer. Particularly, a method for treating a patient in need thereof is described comprising the administration of the pharmaceutical formulations described in the present invention at a dose between 200 mg/70kg and 750 mg/70kg by SC route or IM route. The SC administration of this mAb is performed using an injection volume between 1.3 and 2 mL, and concentrations between 150 and 200 mg/mL. Furthermore, the mAb can be administered at one or more injection sites when higher doses of the mAb are needed. In the case of the IM route, with injection volumes between 1.3 a 5 mL for the 150 mg/mL concentration, doses from 200 to 750 mg/70kg would be achieved. When using a concentration of 200 mg/mL and intramuscular volumes of 1.3-5 mL, doses of 260-1000 mg/70kg would be achieved.

## DETAILED DESCRIPTION OF THE INVENTION

Obtainment of stable highly concentrated formulations of nimotuzumab mAb

The present invention is related to formulations at high concentrations of the mAb nimotuzumab which will also be herein denominated to as hR3. The concentration of nimotuzumab in the formulations described in the present invention is in the range between 100 and 210 mg/ml, preferably in the range between 100 and 180 mg/ml. The nimotuzumab formulations disclosed in this patent application comprise a buffer substance, a surfactant, amino acids, and optionally a carbohydrate. The buffer substance can be histidine or sodium phosphate both in a range between 5 and 30 mM, which allows for the maintenance of the pH of the formulation in a pH range of 6.5 $\pm$ 0.5. The formulations of the present invention can comprise polysorbate 20 or polysorbate 80 as surfactant at a concentration range between 0.02 and 0.06%. The carbohydrates can be sucrose and trehalose at a concentration range between 2 and 6%. Additionally, the amino acids are selected from L-methionine or glycine, or both at a concentration range between 30 and 150mM.

To obtain these formulations, an Amicon Ultra ultrafiltration-diafiltration (UF/DF) system with a cellulose membrane with pore size between 30-50kD can be used. To diafiltrate or concentrate the sample, a centrifugation speed ranging from 900 x g to 1100 x g at temperatures between 2-8 °C can be used. The sample is subjected to a sterilizing filtration, for which the type of filter to be used will be selected depending on the volume of the sample. Another way of preparing the formulations described in the present invention is by means of a laboratory or pilot scale tangential flow filtration system. For this purpose, membranes of 30 and 50kD pore size can be used. The diafiltration of the samples in order to change the buffer is performed by using 10-15 diavolumes of final buffer. To concentrate the samples, the initial volume must be reduced to a volume smaller than the calculated volume that corresponds to the desired concentration. These formulations can be obtained by means of a step by step screening strategy consisting of a first stage in which the best buffers and pH values are selected. In the second stage the most appropriate salts, surfactants and carbohydrates are selected and subsequently in a third stage the best amino acids for the formulation are selected. Another method that can be employed is simultaneous screening, which consists of the use of different types of excipients or combinations thereof according to a factorial design. Using this method, there is the possibility of interaction between more than 2 or 3 excipients in the same formulation.

In another aspect the present invention provides a method for lyophilizing and reconstituting the above- described formulations as well as a method for preparing a stable, isotonic reconstituted formulation. Said method comprises the reconstitution of the lyophilized mixture of the mAb and stabilizers in the buffers described above in such a way that the mAb concentration in the reconstituted formulation is at least 100 mg/mL and up to 210 mg/mL, that is, 3- 5 times greater than the concentration of mAb in the mixture before the lyophilization.

## Determination of the stability of the highly concentrated formulations of nimotuzumab

To verify the most stable formulation variants and the influence of each excipient on stability, a study of the parameters that predict stability is performed by means of dynamic light scattering (DLS). These above-mentioned parameters are the diffusion interaction parameter (kD), the Zeta potential and the aggregation temperature (Tagg). The determination of kD is performed by measuring the diffusion coefficient of the nimotuzumab at a concentration from 12 mg/ml to 2 mg/ml. The Tagg is determined from the initial samples at a temperature ramp from 25 to 76°C and the Zeta potential is determined by measuring the sample at the initial concentration.

Another method used to determine the best formulations is the stress study, where the samples are subjected to a temperature of 50°C in a thermostatic bath from 10 to 20 days and then they are analyzed at different time intervals by means of physicochemical and biological determinations.

Among these determinations is turbidity, which is measured the absorbance of the samples in the UV spectrum between 340 and 450 nm, which allows for the detection of large aggregates. Another example is the determination of particle size by DLS performed by measuring the initial concentration of the intact undiluted sample in order to detect the formation of aggregates or particles in the solution. Viscosity measurement is performing for the case of high concentration samples using a size standard for DLS in a range between 100-500 nm of nominal size, preferably 200 nm of nominal size. Samples are also analyzed by means of SDS-PAGE to determine if there was fragment formation during the stress study. The test was performed under non-reducing conditions using with 7.5% of polyacrylamide gels and Coomassie blue or silver nitrate stains. The determination of monomer purity by means of size exclusion high-performance liquid chromatography (SEC-HPLC) is also carried out.

The biological activity of the mAb was determined by means of the relative potency with respect to the reference material of nimotuzumab and can be measured by flow cytometry or by the inhibition of cell proliferation in a cell line that expresses human Her1.

## Treatment methods

The formulations object of the present invention can be used to treat patients with head and neck, glioma, esophageal, lung and pancreatic tumors. For therapeutic use, said formulations must be administered to a subject bearing the disease as monotherapy or in combination with conventional therapies used for the treatment of tumors such as radiotherapy or chemotherapy, so as to enhance their therapeutic action.

The administration of these formulations is performed by SC or IM route. In the case of the SC route, a minimum sample volume of 1.33 mL is to be used for the 200 mg/70kg dose. For larger doses, the injection volume can be increased up to 2 mL until a maximum of 300/70kg dose is reached. In case the administration of doses greater than 300 mg/70kg is required, a concentration of the mAb greater than 150 mg/mL can be used or the dose can be split and administered at two separate injection sites. The injection sites that can be used for said administration are without being limited to: the deltoid regions, abdominal regions and the frontal region of the thighs. For the dose employed in the clinic which is 200 mg per week, a concentration of 134 mg/mL of nimotuzumab would be enough to administer the treatment. In case higher doses are required, a higher concentration of nimotuzumab, up to 210 mg/mL, can be used. For instance, for the treatment of pancreatic cancer, which requires 400 mg doses, two separate injection sites are to be used.

According to what has been reported in the literature, the maximum IM administration volume allowed is 5mL, therefore, when this route of administration is to be used, the injection volume can be between 1.3- 5 mL lead to doses between 200 and 750 mg/70kg for a concentration of mAb of 150 mg/mL. When 200 mg/mL concentrations are used, the dose can be up to 1000 mg/70kg.

The present invention is further elaborated with the following examples and figures. However, these examples and figures should not be construed as limiting the scope of the invention.

## BRIEF DESCRIPTION OF THE FIGURES

Figure 1. kD values of the hR3 mAb formulation variants determined by DLS.
Figure 2: kD values of the different formulations of hR3 mAb containing amino acids determined by DLS.
Figure 3: Particle size measured by DLS of the hR3 mAb formulations containing amino acids.
Figure 4: Particle size measured by DLS of hR3 mAb formulations at a concentration of 150mg/mL stored at 4°C for 3 months.
Figure 5: Viscosity measured by DLS of the hR3 mAb formulations at a concentration of 150 mg/mL stored at 4°C for 3 months.
Figure 6: Monomer purity measured by SEC-HPLC of the hR3 mAb formulations at a concentration of 150 mg/mL stored at 4°C for 3 months.
Figure 7: Antitumor effect of nimotuzumab mAb administered IV and SC routes at a dose of 50 mg/kg to athymic Balb/c mice.

## EXAMPLES

### Example 1. Screening of formulation excipients for nimotuzumab mAb.

Nine formulation variants were prepared from a factorial design of 4 factors and ½ of fraction, as shown in Table 1, the hR3sol9 variant corresponds to the original formulation of the hR3 mAb. After the solutions were prepared they were filtered through membrane filters, 0.2 μm pore size and stored at 4°C.

Table 1. Composition of the different formulation variants of the hR3 mAb in the screening of excipients.

| Composition of the different variants of the hR3 mAb | | | | | | | | | |
|---|---|---|---|---|---|---|---|---|---|
| | hR3 sol1 | hR3 sol2 | hR3 sol3 | hR3 sol4 | hR3 sol5 | hR3 sol6 | hR3 sol7 | hR3 sol8 | hR3 sol9 |
| 15mM Sodium Phosphate Buffer at pH 6 | + | - | + | - | + | - | + | - | 15mM Sodium Phosphate Buffer at pH 7 |
| 15mM Histidine Buffer at pH 6 | - | + | - | + | - | + | - | + | - |
| NaCl at 75mM | + | + | - | - | - | + | + | - | NaCl at 150mM |
| 5% Sucrose | + | - | - | + | - | - | + | + | - |
| 5% Trehalose | - | - | + | + | - | + | + | - | - |
| Polysorbate 20 at 0.05% | + | - | - | + | + | + | - | - | - |
| Polysorbate 80 at 0.05% | - | + | + | - | - | - | + | + | Polysorbate 80 a 0.02% |
| (+):presence of the excipient in the formulation (-):absence of the excipient in the formulation | | | | | | | | | |

Once the solutions were prepared, the buffer exchange was carried out by means of UF/DF in 50kD pore size, regenerated cellulose membrane, 15mL sample volume Amicon Ultra Centrifugal Filter Units. The samples were centrifuged at 936 x g at a temperature of 4°C. The mAb hR3 was used as a starting product, in a 15 mM sodium phosphate buffer, 150 mM sodium chloride and 0.2 mg/mL Tween 80, pH 7, solution. After preparing the formulations at an Ab 10 concentration of mg/mL, the measurement of the diffusion interaction parameter (kD) was performed by DLS.

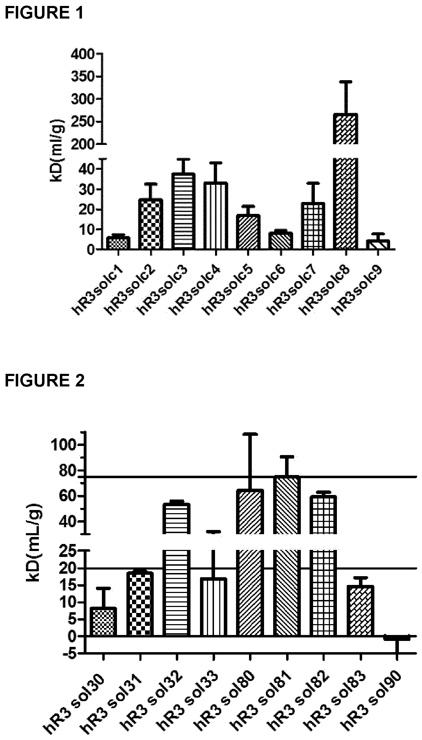

As can be seen in Figure 1 the variants with higher kD values, and therefore with more stable protein-protein interactions and less tendency to aggregate, were the hR3sol8 and hR3sol3 variants, followed by the hR3sol4 variant, which indicates the stabilizing effect of the carbohydrate in the formulation. It was also observed during the course of this test that the kD value of the hR3sol8 variant was much higher than that of the hR3sol3 variant, which suggests the combination of histidine and sucrose has a greater stability than the combination of phosphate with trehalose. In none of the best variants there was presence of NaCl, which shows that it undermines the stability of the hR3mAb. Another of the experiments aimed at verifying which variants were the most stable was the stress test performed at 50°C for 14 days. The samples after being subjected to stress were analyzed by SEC-HPLC to determine the purity of the monomer. Table 2 shows that the variants with the highest monomer purity were hR3sol8 and hR3sol3, which coincides with the results from the kD value measurement. The variants with the lowest monomer purity were hR3sol9 and hR3sol2, that is, the ones that have sodium chloride in their composition, which further confirms previous results that showed the negative effect of this excipient on stability regardless of the type of buffer employed.

Table 2. Monomer purity values, measured by SEC-HPLC, of hR3mAb formulations after being subjected to stress at 50°C for 14 days.

| Monomer purity measured by SEC-HPLC (normalized peak height) | | | | | | | | | |
|---|---|---|---|---|---|---|---|---|---|
| Time (days) | hR3 sol1 | hR3 sol2 | hR3 sol3 | hR3 sol4 | hR3 sol5 | hR3 sol6 | hR3 sol7 | hR3 sol8 | hR3 sol9 |
| 0 | 100 | 100 | 100 | 100 | 100 | 100 | 100 | 100 | 100 |
| 4 | 78.19 | 71.85 | 85.99 | 87.66 | 83.55 | 74.94 | 83.16 | 91.71 | 72.12 |
| 8 | 66.40 | 55.63 | 79.65 | 80.62 | 74.71 | 63.82 | 75.53 | 81.02 | 55.40 |
| 11 | 61.54 | 47.59 | 73.38 | 75.04 | 67.46 | 53.14 | 66.74 | 75.19 | 45.46 |
| 14 | 54.38 | 38.50 | 68.89 | 64.01 | 61.99 | 49.19 | 61.60 | 71.73 | 39.31 |

Based on the previously obtained results, it was concluded that the most stable hR3 mAb formulation variants are those that have some carbohydrate such as sucrose and trehalose in absence of sodium chloride. Furthermore, the combination of sucrose with histidine is considerably more stable than the combination of phosphate with trehalose.

**Example 2. Screening of amino acids for the formulation of hR3 mAb.**

Different solutions were prepared from the two best variants obtained after the screening of excipients and an amino acid (L-arginine, L-methionine, glycine) was added to each one. The preparation method was similar to the one described in Example 1 for the screening of excipients. Table 3 shows the formulation variants of the hR3 mAb that contain the different amino acids.

Table 3. Composition of the different hR3 mAb formulations in presence of amino acids.

| Composition of the different hR3 mAb formulations | | | | | | | | | |
|---|---|---|---|---|---|---|---|---|---|
| | hR3 sol30 | hR3 sol31 | hR3 sol32 | hR3 sol33 | hR3 sol80 | hR3 sol81 | hR3 sol82 | hR3 sol83 | hR3 sol90 |
| 15mM Sodium Phosphate Buffer at pH 6 | + | + | + | + | - | - | - | - | 15mM Sodium Phosphate Buffer at pH 7 |
| 15mM Histidine Buffer at pH 6 | - | - | - | - | + | + | + | + | - |
| NaCl at 75mM | - | - | - | - | - | - | - | - | NaCl at 150mM |
| 5% Sucrose | - | - | - | - | + | + | + | + | - |
| 5%Trehalose | + | + | + | + | - | - | - | - | - |
| Polysorbate 80 at 0.05% | + | + | + | + | + | + | + | + | Polysorbate 80 at 0.02% |

(continued)

| Composition of the different hR3 mAb formulations | | | | | | | | | |
|---|---|---|---|---|---|---|---|---|---|
| | hR3 sol30 | hR3 sol31 | hR3 sol32 | hR3 sol33 | hR3 sol80 | hR3 sol81 | hR3 sol82 | hR3 sol83 | hR3 sol90 |
| 50mM L-Arginine | - | + | - | - | - | + | - | - | - |
| 50mM L-Methionine | - | - | + | - | - | - | + | - | - |
| 50mM Glycine | - | - | - | + | - | - | | + | - |
| (+):presence of the excipient in the formulation (-):absence of the excipient in the formulation | | | | | | | | | |

The results from the kD measurement, performed by DLS, are shown in Figure 2. As can be observed the variants with the highest kD value were hR3sol80, hR3sol81, hR3sol82 and hR3sol32; and out of them the most stable variants were the ones with histidine buffer. It should also be noted that in both buffers the presence of methionine results in high kD values which does not occur with the other amino acids.

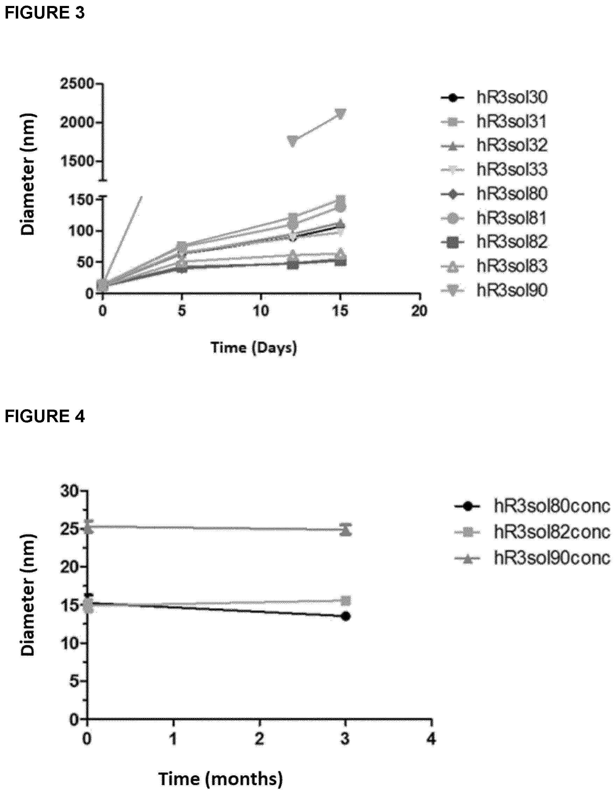

The different formulations were subjected to stress at 50°C for 15 days in a thermostatic bath and the particle diameter was measured by DLS at 0, 5, 12 and 15 days. Figure 3 shows the results from these measurements. As can be seen, the variants with smaller particle diameter, and therefore the ones with less aggregation, were the hR3sol80, hR3sol82 and hR3sol83 variants. This confirms that found that the formulations with histidine buffer are more stable than the ones with phosphate buffer and also that the variants containing L-methionine or glycine are more stable than the ones with L-arginine.

Additionally, Table 4 shows the monomer purity values of the formulations subjected to stress at 50°C.

**Table 4.** Normalized values of monomer purity, measured by SEC-HPLC, of the hR3 mAb formulations at 5 mg/mL in presence of amino acids after being subjected to stress at 50°C.

| Normalized values of monomer purity by SEC-HPLC (normalized peak height) | | | | | | | | | |
|---|---|---|---|---|---|---|---|---|---|
| Time (days) | hR3 sol30 | hR3 sol31 | hR3 sol32 | hR3 sol33 | hR3 sol80 | hR3 sol81 | hR3 sol82 | hR3 sol83 | hR3 sol90 |
| 0 | 100 | 100 | 100 | 100 | 100 | 100 | 100 | 100 | 100 |
| 5 | 78.72 | 74.91 | 78.61 | 79.89 | 87.19 | 71.19 | 86.19 | 82.17 | 64.65 |
| 12 | 65.04 | 57.99 | 63.05 | 63.44 | 73.92 | 52.75 | 76.19 | 60.53 | 51.20 |
| 15 | 58.89 | 52.11 | 58.60 | 61.16 | 65.05 | 44.68 | 72.28 | 49.17 | 39.72 |

Table 4 shows that the variants with the highest monomer purity after being subjected to stress are hR3sol82 and hR3sol80. This result indicates that the variants that have the histidine buffer are more stable than the ones that have the phosphate buffer. It also suggests that the formulation with methionine has more stability than one without amino acids. The formulations with the lowest purity were hR3sol81 and hR3sol90. This suggests that both L-arginine and sodium chloride have a destabilizing effect on the hR3 mAb formulations. According to the previously results the hR3 mAb formulation that has a histidine buffer at pH6, sucrose, polysorbate 80 and L-methionine is the most stable.

These results demonstrate that the hR3sol2, hR3sol6, hR3sol31, hR3sol81 and hR3sol90 variants that are within the claimed range of combinations of patent WO2011080209, are not recommended for the nimotuzumab mAb, since their low stability negatively impacts on their efficacy and the safety of their administration to patients. The above support the concept reported in the literature that the formulations that are stable for one protein are not necessarily stable for another one in reason of the structural differences across proteins.

**Example 3. Shelf-life stability study of the hR3 mAb at high concentration stored at 4°C**

A characterization of the stability of the hR3sol80, hR3sol82 and hR3sol90 variants at a concentration of 150 mg/mL of mAb stored at 4°C was performed. The samples underwent physicochemical analyses of particle diameter and viscosity by DLS and monomer purity analyses by HPLC at 0 and 3 months.

Figure 4 shows the particle diameter results, measured by DLS, of the different variants. As can be observed in the figure, in each of the formulations the diameter remained constant during the 3 months, which indicates the non-occurrence of aggregation in the samples at high concentrations. However, it should be noted that the particle diameter of the hR3sol90 variant was around 25nm at the initial time, which is larger than the one of the other formulations. This suggests this variant has a greater tendency to self-association between molecules with respect to the hR3sol80 and hR3sol82 variants.

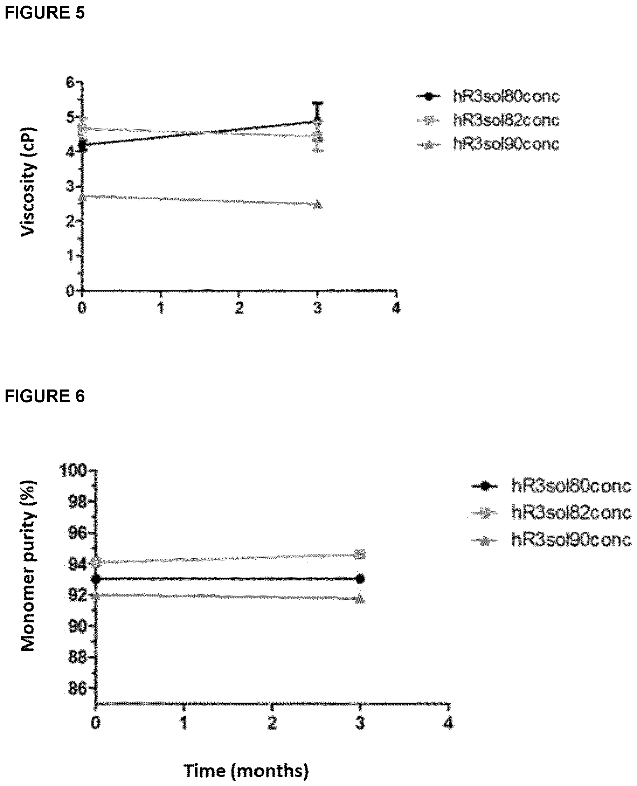

Figure 5 shows the viscosity values, measured by DLS, with a 200 nm particle size standard. In all cases, the viscosity had only very small variations, which indicates that there is no aggregation effect or self-association between molecules. Furthermore, it is very important to highlight that all the values were below 5cP, which is well below the limits established to achieve an adequate injectability and manufacturability (Li Li, Sandeep Kumar et al. Pharm Res (2014) 31: 3161-3178). Also, the viscosity values are lower than the ones reported for other Abs at a concentration of 150 mg/mL.

Figure 6 shows the monomer purity, measured by SEC-HPLC, graph. As can be observed, the purity of monomers did not exhibit considerable changes, which suggests that the formulations of hR3 mAb at a concentration of 150 mg/mL stored at 4°C are stable.

Additionally, the biological activity of the hR3 mAb formulations was determined at a concentration of 150 mg/mL without exposure to stress at time 0 and 3 months. The biological activity was measured by the recognition of the human epidermal growth factor receptor (Her1) by the hR3 mAb by flow cytometry assay and by the inhibition of cell proliferation assay. To perform the flow cytometry assay, $2\times10^5$ H292 cells per well were used and a concentration curve was prepared from 81 $\mu$g/mL with 1/3 dilution, to then determine the mean fluorescence intensity (MFI). The half maximal effective concentration (EC50) was determined from the MFI value and compared to a commercial hR3 reference material to obtain the relative potency. The cell proliferation assay was performed with H292 cells and using cell proliferation reagent WST-1 solution as dye. The absorbance obtained was compared to the commercial hR3 reference material to determine the relative potency. The results from both measurements at time 0 are shown in Table 5 and the ones performed at 3 months after the beginning of the study in Table 6.

**Table 5.** Biological activity of hR3 mAb formulations at a concentration of 150 mg/mL without exposure to stress at initial time.

| Biological activity of the hR3 mAb concentrated at 150 mg/mL at t0 | | | | | |
|---|---|---|---|---|---|
| Sample | Cell Recognition by Cytometry (%) | Mean Fluorescence Intensity(MFI) by Cytometry | | Cell Proliferation Assay | |
| | 3µg/mL | RP Cytometry | CI | RP Cell Proliferation | CI |
| hR3sol80 t0 | 99.11 | 81 | 73-89 | 101 | 91-113 |
| hR3sol82 t0 | 99.04 | 83 | 78-89 | 104 | 78-139 |
| hR3sol90 t0 | 99.11 | 79 | 74-84 | 93 | 59-147 |
| RP: relative potency % with respect to reference commercial hR3 mAb<br>CI: confidence interval | | | | | |

Table 5 shows that the relative potency of the hR3sol80, hR3sol82 and hR3sol90 variants was between 79 and 83% as measured by the flow cytometry assay, which indicates that they maintain their biological activity even at high concentrations. On the other hand, the relative potency values obtained in the inhibition of cell proliferation assay were between 93 and 104%, which confirms the result from the cytometry and demonstrate the samples at high concentration retain their biological activity. It should be taken into account that each value is inside a different confidence interval due to the intrinsic variability in each assay. Despite it the values are inside the range acceptable for each assay.

**Table 6.** Biological activity results of the hR3 formulations at a concentration of 150 mg/mL, stored at 4°C, at 3 months.

| Sample | Cell Recognition by Cytometry (%) | Mean Fluorescence Intensity(MFI) by Cytometry | | Cell Proliferation Assay | |
|---|---|---|---|---|---|
| | 3 µg/mL | RP Cytometry | CI | RP Inhibition | CI |
| hR3Sol 80 3M | 93,93 | 81 | 56 - 117 | 77 | 66 - 89 |

(continued)

| Sample | Cell Recognition by Cytometry (%) | Mean Fluorescence Intensity(MFI) by Cytometry | | Cell Proliferation Assay | |
|---|---|---|---|---|---|
| | 3 μg/mL | RP Cytometry | CI | RP Inhibition | CI |
| hR3Sol 82 3M | 87,85 | 46 | 42 - 49 | 45 | 35 - 57 |
| hR3Sol 90 3M | 93,76 | 78 | 72 - 84 | 86 | 69 - 107 |
| RP: relative potency % with respect to the reference commercial hR3 mAb CI: confidence interval | | | | | |

Table 6 shows the biological activity results of the hR3 formulations at a concentration of 150 mg/mL, stored at 4°C, at 3 months. As can be noted the samples with the highest relative potency value were hR3sol80 and hR3sol90, whose values were between 78 and 81% in the recognition by cytometry assay and between 77 and 86% in the inhibition of proliferation assay.

**Example 4. Stress study at 50°C of the best formulation variants of the hR3 mAb at a concentration of 150 mg/mL.**

In the formulation variants hR3sol80, hR3sol82 and hR3sol90, the hR3 was concentrated up to 150 mg/mL by UF/DF in 50kD pore size, regenerated cellulose membrane, 15mL sample volume Amicon Ultra Centrifugal Filters Units.. The samples were centrifuged at 936 g at a temperature of 4°C. Once the concentration of 150 mg/mL was reached, the samples were filtered with 0.2 μm pore size filters and placed in a thermostatic bath at 50°C. A sampling of the different formulations was performed on days 0, 4, 10 and 15 to measure physicochemical parameters. Table 7 shows the results from the monomer purity measurement performed on the different sampling days.

**Tabla 7.** Normalized values of monomer purity of the hR3 mAb formulations at a concentration of 150 mg/mL after being subjected to stress at 50°C for 15 days.

| Monomer purity results of hR3, measured by SEC-HPLC, at a concentration of 150 mg/mL, after being stressed at 50°C (normalized peak height) | | | |
|---|---|---|---|
| Time (days) | hR3sol80conc | hR3sol82conc | hR3sol90conc |
| 0 | 100.00 | 100.00 | 100.00 |
| 4 | 45.34 | 58.99 | White precipitate |
| 10 | Yellow gel | 43.94 | White precipitate |
| 15 | Yellow gel | Yellow gel | White precipitate |

As can be seen in Table 7, after 4 days of stress, the hR3sol90 variant had completely precipitated forming a white solid while the hR3sol80 variant had a monomer purity of 45.3% and the hR3sol82 variant a monomer purity of 58.9%. As can be concluded from this result, the most stable formulation at high concentrations is hR3sol82, whereas the least stable one is hR3sol90. This result coincides with the ones obtained at low concentration. At 10 days of stress, the hR3sol80 variant forms a yellow gel while the hR3sol82 variant continues in solution with 43.9% monomer purity. On the sampling performed at day 15, the hR3sol82 variant also forms a yellow gel. The previous results demonstrate the hR3sol82 formulation that has L-methionine in the composition is more stable at high concentrations than the variants that lack this amino acid. These results suggest that in the long term the most stable formulations would be hR3sol80 and hR3sol82, whereas the hR3sol90 formulation would be the least stable one. Table 8 shows the percentage of monomer and dimers in the samples at a concentration of 150 mg/mL without stress exposure.

Table 8. Monomer purity of the hR3 mAb formulations at a concentration of 150 mg/mL without stress exposure.

| Purity of hR3 at a concentration of 150 mg/mL at t0 measured by SEC-HPLC (%) | | | |
|---|---|---|---|
| Molecular Species | hR3sol80conc | hR3sol82conc | hR3sol90conc |
| Monomer | 93.03 | 94.11 | 92.02 |
| Dimer | 6.30 | 5.29 | 7.17 |
| Aggregate | 0.56 | 0.45 | 0.79 |
| Fragment | 0.099 | 0.015 | 0.010 |

As can be observed in the table the hR3sol82 formulation is the one with the highest monomer percentage (94.1%) and the one with the lowest dimer percentage (5.2%), which demonstrates this formulation has a trend towards a reduction in the number of associations between the molecules contained in it that favors stability. The hR3sol80 and hR3sol90 variants have monomer percentages of 93.0% and 92.0% respectively.

The biological activity of the hR3 mAb formulations at a concentration of 150 mg/mL was also determined by flow cytometry before subjecting them to stress at 50°C and by means of the inhibition of cell proliferation assay. To perform both determinations the same methodology described in Example 3 was used.

**Example 5 Bioavailability of nimotuzumab mAb administered by SC route at different dose levels to Balb/c mice.**

Taking into account that the same Ab and administration route are used and that the changes in the formulation components do not significantly influence the pharmacokinetic behavior of the Ab, the bioavailability study was performed with the hR3sol90 variant. To perform this assay, female Balb/c mice of approximately 20 g of weight were used. The animals were divided into 4 groups that received 4 different dose levels (25, 50, 100 and 200 mg/kg of weight) of the hR3sol90 formulation by SC. Before its administration the mAb was labeled with isotope Iodine 125 ($^{125}$I). Then the blood of each individual mice was extracted and the radioactivity was measured, which is proportional to the Ab concentration in the blood. Table 9 shows the bioavailability values obtained with the different dose levels compared to an IV dose of 25 mg/kg. The following formula is used for the calculation of bioavailability (F):

$$F = ( SC\text{-}AUC / IV\text{-}AUC)*( IVd / SCd)$$

where:

SC-AUC: area under the curve of the SC route
IV-AUC: area under the curve of the IV route
IVd: IV dose
SCd: SC dose

For the IV route the bioavailability is 1, as 100% of the parent drug enters the general circulation.

Table 9 Bioavailability of the nimotuzumab mAb administered by SC route at different dose levels.

| Absolute bioavailability calculation based on the area under the curve from 0 to infinity of the nimotuzumab mAb administered by SC route | | | | |
|---|---|---|---|---|
| Volume administered (mL) | total Mass administered (mg) | SC dose in mg/kg | Area under the curve from0-∞ (μg/ml*h) | Bioavailability (%) |
| 0.2 | 0.5 | 25 | 15153 | 83.7 |
| 0.2 | 1.0 | 50 | 30813 | 87.2 |
| 0.2 | 2.0 | 100 | 54264 | 78.7 |
| 0.2 | 4.0 | 200 | 124280 | 88.0 |
| IV dose =25 mg/kg | | | | |

The results in Table 9 indicate that in all cases the bioavailability was above 75% which is acceptable for this type of administration route. It should be noted that even though the bioavailability practically does not increase as the dose increases, the total mass of the mAb and the area under the curve do increase. If these results are extrapolated to humans, for example, in the case of the SC dose of 100 mg/kg, that implies that, even if the bioavailability is 78.7%, the total mass of mAb that would reach the bloodstream would be 630 mg, which is more than three times the dose used in the clinic (200 mg).

**Example 6. Antitumor Effect of nimotuzumab mAb administered by IV and SC routes at a dose level of 50 mg/kg in athymic Balb/c mice.**

Female athymic Balb/c mice weighing approximately 20 g were divided into three groups of five animals each. All groups received 2x106 A431 cells on day zero and on days 10, 12, 14 and 16, Group 1 was administered 1mg of total hR3sol90 formulation mass by the SC route, Group 2 received 1mg of total nimotuzumab mAb mass by the IV route and the control group, Group 3, received phosphate buffered saline.

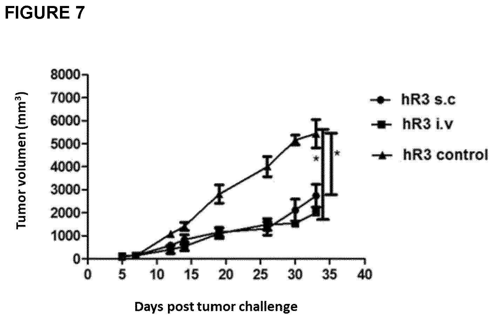

Figure 7 shows the tumor volume in the mice that were administered nimotuzumab by the SC route and by the IV route were similar. This demonstrates that there were no differences in the efficacy of the treatment when using these two routes despite of the fact that the absorption rate and pharmacokinetic characteristics of each route are different. On the other hand, the control group had a much higher tumor growth rate, which was even significantly different from the growth rate of the other two groups. This demonstrates the efficacy of the administration of nimotuzumab mAb by SC route.

**Example 7. Purity of the lyophilized nimotuzumab mAb at a concentration of 150 mg/mL.**

The hR3sol80, hR3sol82 and hR3sol90 formulations were lyophilized at a concentration of 150 mg/mL and a volume of 500 μL. For this, a laboratory lyophilizer at a temperature of -30°C was employed, a vacuum was made for 30 hours until a dry powder was obtained. Then the formulations were reconstituted in 500 μl of ultrapure water until the initial concentration was reached and a transparent, particle-free solution was obtained.

Table 10 shows the purity data results from the different nimotuzumab mAb formulations after being lyophilized and subsequently reconstituted.

**Table 10.** Purity of nimotuzumab mAb after being lyophilized and reconstituted.

| Purity of the nimotuzumab mAb after being lyophilized and reconstituted at a concentration of 150 mg/mL | | | |
|---|---|---|---|
| Samples | Monomer (%) | Dimer (%) | Monomer +Dimer (%) |
| hR3sol80 | 94.18 | 5.73 | 99.91 |
| hR3sol82 | 98.8 | 1.17 | 99.97 |
| hR3sol90 | 93.65 | 5.89 | 99.54 |

As can be observed, the hR3sol82 formulation after being lyophilized and reconstituted reached a monomer purity of more than 98% whereas the hR3sol80 variant reached a monomer purity of around 94% and the hR3sol90 variant of more than 93%. It should also be noted that the percentage of dimers changes depending on the type of formulation. Additionally, the table shows the purity of monomer plus dimer of these lyophilized and reconstituted formulations was in all cases above 99%, which indicates that the nimotuzumab mAb does not lose its stability despite being stressed during the lyophilization process. Can be noted that nimotuzumab mAb has a balance between monomers and dimers due to the characteristics of this mAb, this balance can vary depending on the type of interactions that occur in the solution.

**Claims**

1. a highly concentrated and stable pharmaceutical formulation of the monoclonal antibody (mAb) nimotuzumab **characterized in that** it comprises:

   a) the nimotuzumab mAb at a concentration within the range from 100 to 210 mg/mL,
   b) a buffer substance at a concentration within the range from 5 to 30 mM, at pH range of 6.5 ± 0.5,
   c) a surfactant within the range from 0.02 to 0.06 %,
   d) an amino acid or mixture thereof within the range from 30 to 150mM and

e) optionally a carbohydrate as stabilizing agent within the range from 2 to 6 %.

2. The pharmaceutical formulation according to claim 1 wherein the buffer substance is selected from the group comprising:

   - histidine buffer and
   - sodium phosphate.

3. The pharmaceutical formulation according to claim 1 wherein the surfactant is selected from the group comprising:

   - polysorbate 20 and
   - polysorbate 80.

4. The pharmaceutical formulation according to claim 1 wherein the amino acid is selected from the group comprising:

   - L-methionine and
   - glycine.

5. The pharmaceutical formulation according to claim 1 wherein the carbohydrate is sucrose.

6. The pharmaceutical formulation according to any of claims 1-5 in its liquid form.

7. The pharmaceutical formulation according to any of claims 1-5 in its lyophilized form.

8. The pharmaceutical formulation according to claim 6 **characterized in that** it has a viscosity $\leq$ 5cP at a concentration of 150 mg/ml.

9. Use of the pharmaceutical formulation of any of claims 1-8 for the treatment of cancer.

10. A method for treating a patient in need thereof comprising the subcutaneous administration of the pharmaceutical formulations of any of claims 1-8 at a dose level between 200 mg/70kg and 400 mg/70kg using an injection volume between 1.3 and 2 mL, and wherein the dose of the pharmaceutical formulation can be split and administered at two or more separate injection sites.

11. A method for treating a patient in need thereof comprising the intramuscular administration of the pharmaceutical formulations of any of claims 1-8 at a dose level between 150 mg/70kg and 1000 mg/70kg using an injection volume between 1.3 and 5 mL.

**FIGURE 1**

**FIGURE 2**

**FIGURE 3**

**FIGURE 4**

**FIGURE 5**

**FIGURE 6**

**FIGURE 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9345661 B
- US 10280227 B
- CN 107898756 A
- WO 2011080209 A

### Non-patent literature cited in the description

- **MATEO, C. et al.** *Immunotechnology,* 1997, vol. 3, 71-81
- **CROMBET, T. et al.** *J Clin Oncol.,* 2004, vol. 22, 1646-1654
- **RAMOS, T. et al.** *Cancer Biol Ther.,* 2006, vol. 5, 375-379
- **MACDONALD, T. et al.** *Neuro Oncol.,* 2011, vol. 13, 1049-1058
- **RAMOS-SUZARTE, M. et al.** *Cancer Biology & Therapy,* 2012, vol. 13, 600-605
- **GALLUZZI, L. et al.** *OncoImmunology,* 2012, vol. 1, 28-37
- *Revista Cubana de Farmacia,* 2012, vol. 46 (3), 379-380
- **MICHAEL F. HALLER.** *Pharmaceutical Technology,* 2007, vol. 31 (10), 118-132
- **VIOLA M, SEQUEIRA et al.** *J Control Release,* 2018, vol. 286, 301-314
- **HALLER MF.** *Pharmaceutical Technology,* 2007, vol. 31 (10), 118-132
- **JING-FEN JIN et al.** *Patient Preference and Adherence,* 2015, vol. 9, 923-942
- **SHPILBERG, O. ; C. JACKISCH.** *British Journal of Cancer,* 2013, vol. 109, 1556-1561
- **MANNING MC et al.** *Advances in Protein Chemistry and Structural Biolog,* 2018, vol. 112, 1-59
- **VIOLA M ; SEQUEIRA J et al.** *J Control Release,* 2018, vol. 286, 301-314
- **LI LI ; SANDEEP KUMAR et al.** *Pharm Res,* 2014, vol. 31, 3161-3178